# EUROPEAN PATENT APPLICATION

(11) **EP 1 283 479 A1**
(43) Date of publication of application: **12.02.2003**
(21) Application number: 01912231.6
(22) Date of filing: 09.03.2001
(51) Int. Cl.: G06F 17/30

(54) **METHOD FOR PROCESSING INFORMATION ABOUT CHEMICAL SUBSTANCE**

(30) Priority: 13.03.2000 US 188682 P; 14.03.2000 JP 2000069869
(71) Applicant: CHUGAI SEIYAKU KABUSHIKI KAISHA, Tokyo, 115-8543 (JP)
(72) Inventor: NAGASHIMA, Renpei, Toshima-ku, Tokyo 170-0002 (JP)
(74) Representative: VOSSIUS & PARTNER
(86) International application number: JP0101864
(87) International publication number: WO01069440

(57) **Abstract**

This invention provides information libraries of chemical substances (referred to as "chenes") which interact with various biological systems. Also, disclosed are efficient methods to construct such libraries and data sharing systems which enable efficient utilization of such libraries. Furthermore, this invention provides databases which accommodate and maintain information libraries relative to such chenes; methods and systems to construct such databases by accumulating those pieces of information which concern chenes interacting with various biological systems; methods and systems to enable a client to obtain desired information by searching the constructed databases; methods and systems to transmit to such a client the desired pieces of information concerning chenes that are housed in such database; tangible electronic means to record and make use of such systems and database; and an apparatus to enable construction and search of such database and/or transmission of desired information to such a client.

## Description

### Technical Field

This invention relates to information libraries of chemical substances (which are termed "chenes") which interact with various biological systems, efficient methods to construct such libraries, and data sharing systems which enable efficient utilization of such libraries. Furthermore, this invention includes databases which accommodate and maintain information libraries relative to such chenes; methods and systems to construct such databases by accumulating those pieces of information which concern chenes which interact with various biological systems; methods and systems to enable a client to obtain the desired information by searching such constructed databases, methods and systems to transmit, to the client, desired information concerning chenes that are housed in such databases; tangible electronic means to record and make use of such systems and databases, and apparatus to enable construction and search of such databases and/or transmission of desired information to such a client.

### Background Art

Research for discovery and development of new drugs begins with exploration, identification, characterization, and validation of drug targets. Such drug targets are frequently selected from specific cell surface receptors, adhesion molecules, enzymes, agents that participate in intracellular transmission of information (often called signal transduction), intranuclear receptors, transcription factors, cytokines, chemokines, intercellular substances, etc. Sometimes, specific tissues, organs, and whole animals (that are called experimental disease models, when appropriate) are chosen as direct drug targets. Thereafter, screening methods are devised for such selected drug targets (those applied recently include automation, robotics, high-throughput settings, miniaturization, nanometrics, microfluidics, etc.). Chemical libraries from various sources are screened for selected biological activity or non-activity by use of such screening methods (wherein technology of combinatorial chemistry is utilized to construct such chemical libraries when appropriate), and so-called "hits" are identified. So-called "lead compounds" are generated by applying various algorithms to such hits, and lead compounds are optimized to yield a single or multiple candidate compound(s) by considering so-called "drug-likeness" (for example, as described in Clark, D.E. and Picket, S.D. Drug Discovery Today (2000) 5: 49-58) and biological properties as observed in experimental animal systems. Such candidate compound or compounds are formulated in the form of an appropriate preparation (now called drug product) and tested for safety and efficacy in humans. The size of target market and competitive advantage as well as disadvantage of a candidate drug product or products are studied. Then, when all existent and foreseeable hurdles are recognized to be overcome by a candidate drug product, an application is filed with a drug regulatory agency of the respective country or region (such as EC) for market release approval of the candidate drug product.

Only when a compound is approved by drug regulatory agency of respective country or region, it is placed on sale in the market in the form of drug product as a remedy for a cure and treatment (or even prophylaxis) of a particular disease with associated symptoms. (While the term "compound" is used herein, this term covers a wide range of agents and includes biotechnology products like proteins, enzymes, antibodies, etc. (those not obtained by chemical synthesis, and natural products, such as, antibiotics.) As is clear from this description of drug research and development, there exist an enormous number of hurdles for a drug product to obtain approval prior to its reaching a particular market. In fact, one can say that such hurdles are "innumerable."

Data from seventeen representative Japanese pharmaceutical companies during the period from 1992 to 1996 discloses the rate of compounds that were successfully developed and that finally reached the market (success rate of drug development) was one in 6,053 compounds that were synthesized (or obtained) and tested (DATA BOOK, 1999, Japan Pharmaceutical Manufacturers Association). Pharmaceutical industries in the United States of America and European countries also face similar obstacles in drug research and development. Accordingly, while an enormous number of compounds are synthesized (or prepared by other means) and tested, most of these compounds are dropped during research and development process. Herein, these compounds are termed "unsuccessful compounds."

Why do we see so many unsuccessful compounds? One of the major factors lies in the difficulty in obtaining an appropriate list of hit compounds that would reasonably enable generation of lead compounds. However, even if this difficulty is overcome there is little probability of encountering the desired lead compounds. This would be so even if a validated drug target is identified and recent technologies for drug research as high throughput screening (HTS), structure (/substrate)-based drug design (SBDD), and combinatorial chemistry are employed. While this is partly due to the limitation in the number and diversity of chemical substances (chenes) in the library of compounds that are proprietary and/or available to a company, a much greater problem arises from the lack of the science which serves to generate lead compounds from a given list of hit compounds and optimize lead compounds to yield a desired drug product. In fact, the following tendency is widely recognized: since the introduction of HTS in drug research, a considerable number of compounds synthesized in pharmaceutical houses have become greater in size to have molecular weight exceeding 500; their lipophilicity (as determined usually by octanol/water partition coefficient) have increased tremendously, and their solubility in water have decreased as low as that of insoluble substances; as a result, lead compounds are as such difficult to be absorbed from the digestive tract (Lipinsky, C.A., et al. Advanced Drug Delivery Reviews (1997) 23: 3-25), and it is impossible to find clues to improve and optimize their "drug-likeness" and other biological characteristics. Furthermore, for example, while many kinds of kinases are known to work in intracellular signal transduction pathways, the science is lacking that teaches how to distinguish one kinase working in a specific manner from another that works in a different manner and, more importantly, how to interfere with its action with a "drug-like" chene specifically. As a result, pharmaceutical houses are currently forced to repeat so-called random screenings with little confidence or assurance for success in finding a set of good hits or leads. Still another example concerns chenes that modulate protein-protein interactions. Some people believe that these chenes need to be much larger than those that modulate small molecule ligand-protein interactions. While these large molecule chenes are suitable to interfere with the target protein-protein interactions, they are unable even to pass the cell membrane and reach inside the cell where such interference is desired. Protein-protein interactions are seen frequently in intracellular signal transduction and among transcription factors, and many pharmaceutical companies have selected as a drug target. Unless science concerning chene-biological molecule interactions is founded and advanced, and unless some ways and means in drug designing methodology are discovered, based upon science of such scope, it would be hopeless to discover a drug that would modulate protein-protein interactions and accordingly, that would interfere with signal transduction pathways and interactions among transcription factors.

Recently, a group of relatively small molecule chenes have been discovered which act in an allosteric manner after attachment and normalize the function of a mutant p53 protein that lacks the ability of the normal p53 protein to bind a specific DNA sequence (Foster, B.A. et al. Science (1999) 286: 2507-2510). This is an example of chenes which interfere with nucleic acid-protein interaction which may share some degree of similarity to protein-protein interactions in the sense that interacting molecules are large in both the cases. However, this discovery was made because of random screening of as many as 100,000 compounds and should not be viewed as a success example resulting from science. Clearly, it is necessary to develop science in order to predict what chenes are effective in producing such interference.

There is also a lack of science which serves to identify an appropriate drug target. As already mentioned, identification of a drug target is the first step in drug research and development. The lack of science in this respect is largely due to no or poor scientific information concerning interactions between chenes and biological systems. Until development of such science leads to understanding of characteristics of chenes that is desirable as drug compounds, it is impossible to reasonably define the characteristics of a corresponding drug target and retrospectively, based on such characteristics, to construct a legitimate methodology to discover a most appropriate drug target. In other words, unless a person knows a thing the person wants to find or obtain, it is impossible to devise a method to find or obtain the thing.

One of the most important reasons for seeing so many unsuccessful products is the lack of disclosure of data arising from in-house processes of research and development relative to drug products. While information and data concerning successful products and related compounds are frequently disclosed, that concerning unsuccessful compounds is retained internally and hence totally unavailable even to people in academia and other pharmaceutical houses.

Many of pharmaceutical companies recently purchase commercially available chemical libraries, use them in screening for biological activities, and obtain various data on chenes contained in such libraries. Data, however, are not disclosed and are destined to remain buried within those companies except for findings on certain chenes which have led to successful products. It often happens that different pharmaceutical companies screen chenes of the same commercially available libraries for the same biological activity and share the same failure and error in expectation for success. This constitutes duplication of developmental efforts and costs, and imposes burdens of waste on pharmaceutical companies. Such waste is found not only in pharmaceutical industries but also in other industries.

It is emphasized herein that science is founded and advanced because of accumulation of information and data, i.e., facts. Without observations and facts obtained, no science can be founded and advanced. The lack of disclosure of information and data on most of the chenes studied in industries (except for those related to successful products) causes serious limitation to construction and advancement of science concerning chene-biological system interactions. The lack of opportunities for sharing information and data causes not only duplicative waste of efforts and costs in industrial research and development as mentioned in the preceding paragraph, but also gravely hinders the development of an important branch of science. Science has its highest value in predictive power based on established scientific rule(s). Waste of efforts and costs is avoidable if a rule(s) is/are discovered and proven by scientific studies. For example, science can predict as to what target will be appropriate (conversely, what target is inappropriate), what kind of chenes are desirable for a selected target, and how such desirable chenes can be detected. These kinds of principles apply widely to chene-related industries.

### Disclosure of the Invention

This invention was made in view of the above-mentioned situation and based on considerations given thus far. The objective of this invention is to systematically relate a particular chene with its structural, physical, and chemical profiles and/or its functional profiles. By achieving this objective, this invention provides useful information for desired chemical substances (chenes) which display a profile of desired mode of interactions with the biological system.

This invention further provides data sharing systems as a preferable embodiment of effectively constructing information libraries of such chenes and efficiently utilizing such libraries. This invention, by providing such data sharing systems, enables collection of information rich in quantity and variety, accelerates the advancement of related science and technology, and teaches how to avoid duplicative research and duplicative investments. As a result, this invention promotes rapid progress of research and development in industries.

In summary, this invention includes information libraries of chemical substances ("chenes") which interact with various biological systems, efficient methods to construct such libraries, and data sharing systems that enable efficient utilization of such libraries. Furthermore, this invention includes databases which accommodate and maintain information libraries relative to such chenes, methods and systems to construct such databases by accumulating those pieces of information which concern chenes which interact with various biological systems, methods and systems to enable a client to obtain desired information by searching through such constructed databases, methods and systems to transmit to such a client desired pieces of information concerning chenes that are housed in such databases, tangible electronic means to record and make use of such systems and databases, and an apparatus to enable construction and search of such databases and/or transmission of desired information to such a client.

### Information library of chenes

The "information library of chenes" of this invention is a library consisting of certain chenes selected from chemical substances present in the world (chenome) and accommodates both structural, physical, and chemical profiles and functional profiles of chenes.

"Chenome" and "chene" of this invention are concepts in "chenomics" similar to the concepts of "genome" and "gene" in "genomics".

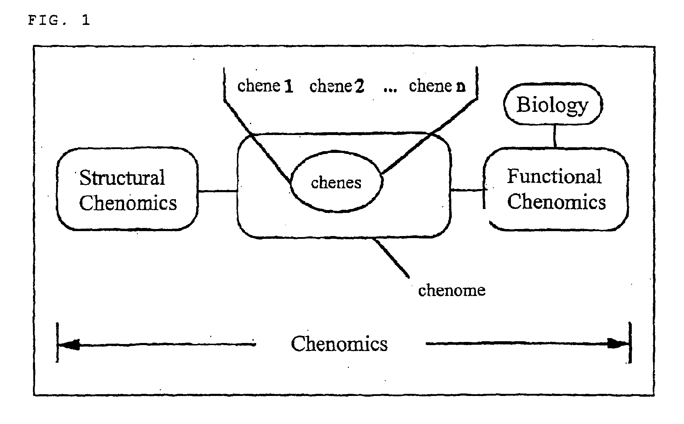

Genomics is a branch of science that studies the genome. The "human genome" means both the sum of all genetic information possessed by the human cell and the whole of genetic materials carried by humans. While the genetic material is thought to be typically represented by DNA molecules, it should be remembered that RNA molecules are genetic materials in RNA-viruses and that prions are genetic materials in broad sense in prion disease. Approximately 3,000,000,000 (10⁹) base pairs of human genome means 3x 10⁹ base pairs which form all of DNA molecules consisting of the 22 pairs of human autosomal chromosomes plus a pair of sex chromosomes. A gene is the most important component of the genome. It is a specific sequence of DNA coding for a particular protein (polypeptide) and genetically it represents the unit of intergenerational transmission, recombination, and function (Ott, J. Analysis of Human Genetic Linkage (1999), 3rd Edition, p 3, The Johns Hopkins University Press). On the other hand, "chenomics" is a branch of science whose purpose is to study the relationship of all chemical substances (chenes) which can exist in the world with substances which belong to the biological system, as well as with the biological system itself. "Chenomics" includes structural chenomics and functional chenomics. Due to the importance of interactions between chemical substances and the biological system, "structural chenomics" is herein defined as a sub-branch of chenomic science which concerns those chenes which have certain significance with respect to interactions with the biological system, or which can be present, with or without known significance in the biological system. In contrast, "functional chenomics" is' defined as another sub-branch of chenomic science which studies the aspect of functions of the chene that are exerted on the biological system and, vice versa, functions of the biological system as exerted on the chene. Fig. 1 illustrates these concepts as mentioned above and emphasizes the relationship of chenomics with biology through functional chenomics.

Genomics, which refers to the study of the structure and the function of the genome, is an integration of structural genomics (which concerns the structure of DNA molecules that carry the genome) and functional genomics (which concerns the function of each of the structural elements of the genome). Functional genomics is the field of study which is expected to grow rapidly especially since the determination of the genome sequence of various organisms (including that of humans) is ongoing,. Such expectation for the rapid growth of functional genomics is based on the increasing trend of sharing the large masses of information among researchers and institutions. This has been made possible by the development of computational science and the global expansion of the Internet. Such science that deals with information is called informatics and is being developed very rapidly. On the other hand, while communication science is closely related to informatics, it has yet to grow to the point of satisfying the current and future needs in informatics. The significance of this invention also lies here.

Information in genomics is not always shared publicly and openly. The venture companies, such as, Celera, Incyte, HGS, and Gene Logic adopt a closed network system formed among specific private companies (sometimes called "subscribers" who pay fees) and a limited number of academic collaborators.

This invention provides a system for sharing of "chenomic" information that is similar to the one developed in genomics. (The term "chenomic" is the adjective meaning "pertaining to chenomics.") However, it is not possible to construct an information sharing system in chenomics by simply imitating the genomic counterpart. In contrast to genomics, chenomics has, unique demands to be satisfied and unique problems to be solved. This invention has been made by considering these situations, by satisfying those demands, and by solving such problems. This invention is therefore expected to serve the future advancement of science and technology of chenomics-related fields in an effective and efficient manner.

The term "chenome" as used herein means the sum of all chemical substances that are and can be present in the world, and corresponds to the term "genome" as used in genomics. (The chenome includes naturally occurring chemical substances as well as artificially produced chemical substances.) According to one theory, the number of different chemical molecules which are and can be present in the space occupied by chemical substances (referred to as the "chenome space") is thought to be more than 10¹⁸ (Clark, D.E. and Picket, S.D. Drug Discovery Today (2000), 5: 49-58). This is to be compared with the 3x 10⁹ base pairs of the human genome.

A chene as used herein refers to a chemical substance that is an important component of the chenome and corresponds to a gene, the most important component of the genome. A chene is a molecule and can be large (such as a polymer), small (like H₂ and glycerol), or intermediate in molecular weight. However, in principle, the concept of chene does not include, those molecules which form the environment (referred to as "environmental molecules") such as molecular nitrogen and oxygen in the atmosphere and solvent molecules in a solution. It is to be noted that molecular oxygen or nitrogen present in water are regarded as a chene since it is not defined as an environmental molecule. Hence, the definition of a chene depends on the situation.

There is a variety of parameters to describe the structural, physical, and chemical profile of a chene. Such profile is the subject of study in structural chenomics. The following are examples of parameters and expressions to describe such a profile. Structural formula itself along with steric characteristics is one of the most important parameters. Molecular weight is a representative parameter that indicates the size of a chene. Polarity and dipole moment are examples of parameters representing electromagnetic characteristics of chenes. The form of a chene can be expressed in a variety of ways, for example, linear (including, string-like which implies flexibility), plane, disk-like, cubic, globular, conic, etc. Flexibility of the form itself also must be taken into consideration. Elongation, shrinkage, free movement, vibration, and rotation are examples of such flexibility. Furthermore, when a chene is locally focused on, local form, electron density distribution, polar surface distribution, hydrogen bonding site distribution, hydrophobic surface distribution, van der Waals force distribution, and so forth become important. Flexibility is to be considered also in this aspect. In addition to expressions such as α-helix and β-sheet, to describe structural features, it is possible to employ figurative expressions like chain, bridge, pocket, groove, door, broom, brush, key, keyhole, wedge, etc. These figurative expressions are particularly useful for proteins, enzymes, peptides, nucleic acids, lipids, and carbohydrates as the concept of a chene includes these molecules. Words like being buried, penetrate, perforate, and pierce may also be used. Structural chenomics also deals with physical and chemical characteristics that arise from interactions with non-biological systems, such as, solubility and stability in water or other aqueous and organic solvents. Structural features which influence these physical and chemical characteristics are also dealt with by structural chenomics. It is desired that the method for synthesis or preparation of a chene to be included in structural chenomics data.

The functional profile of a chene is the subject of study in functional chenomics. The functional characteristics of a chene can be represented in various ways, depending on the biological system of interest (e.g., species of the organism; individual within that species; organ; tissue; cell; cell membrane; nuclear membrane; cytosol; intracellular organelles such as nucleus, mitochondria, ER, and Golgi apparatus; cytoplasmic units such as ribosome; units of each intracellular organelles; and biological molecules such as proteins, nucleic acids, enzymes, peptides, lipids, and carbohydrates). "Death" is one of those examples and includes the death of an individual organism (including man and animal), that of an organ, that of a tissue, and that of a cell (which is usually expressed by the term "cytotoxicity"), etc. As described above, a chene can cause death at various levels of the biological system. Particularly at the cellular level of death, the distinction between necrosis and apoptosis is well known. The same expression can have different meanings, depending on the level of the biological system in consideration; for example, the term "degeneration" can mean denaturation such as unfolding of proteins and nucleic acids at molecular level, while the same term is used for description of pathological changes of a cell or a tissue (in similar meanings such as departure from nature but) in different meanings such as fatty degeneration. Not only "death" and "degeneration" but also other functional characteristics of a chene, regardless of whether it causes a positive (beneficial) or negative (harmful) change in the biological system, are described by morphological, biochemical, and functional expressions. (In microbiology, the term "phenomics" has recently been used to describe morphological, biochemical, and functional characteristics of a microorganism, which is means for classification in classic taxonomy. On the other hand, means for classification in modern microbiological taxonomy is "genomics", in which the DNA sequence coding for ribosomal RNA is most frequently used.) Conversely, changes in a chene resulting from its interaction with the biological system are also included in the functional profile of a chene. Examples of such active (chene on biological system) and passive (biological system on chene) phenomena and properties include binding, association, coupling, affinity, adhesion, agglomeration, aggregation, grouping, repulsion, diffusion, dispersion, bridging, linking, transport, migration, movement, stimulation, activation, suppression, inhibition, avoidance, depolarization, excitation, emotion, affection, desire, volition, esterification, oxidation, reduction, alkylation, dealkylation, cell membrane permeability, bioavailability, gastrointestinal absorbent, blood-brain barrier permeability, susceptibility to drug-metabolizing enzymes, elimination, and excretion. (This is not meant to be an exhaustive list of examples.) The particular biological system where a change is caused by a chene, such as whole body, organ, tissue, cell, cellular organelle, intracellular compositional unit or biological molecule, is to be included in the functional profile of a chene. Examples of passive change of a chene are hydrolysis by esterase when it is an ester and hydroxylation by drug-metabolizing enzyme when it contains an aromatic ring in its structure. Accordingly, the particular biological system that has caused a change in a chene, such as whole body, organ, tissue, cell, cellular organelle, intracellular compositional unit or biological molecule, is to be included in the functional profile of a chene. Note herein that a chene can include not only an exogenous molecule but also an endogenous molecule for the biological system. When a chene is given *in vitro* or *in vivo* at a certain concentration or dose level, tissue or cell demonstrates a characteristic expression profile as indicated by quantitative changes in different mRNA species. Along with its time course, this is an important functional profile of a chene. Similarly, the proteomic expression profile as determined at the protein level (in lieu of the mRNA level) serves to characterize a chene in its functionality. The presence or absence of biological activity of a chene on a drug target and the degree of that activity (if it is present) as determined by appropriate assays is one of the most important data expressing the functional profile of a chene. These kinds of activities are often expressed as the value of EC50, IC50, ED50, etc. In addition, the data obtained from human clinical studies of a chene and used to see if there is an improvement in a particular disease or an associated symptom constitute another important set of information characterizing the functional profile of that chene. Likewise, the functional profile of a chene as it relates to toxicity and safety is important. Examples of expressions for these aspects include cytotoxicity, toxicity in animals (often-expressed in terms of LD50 and macroscopic or microscopic pathologic changes), mutagenicity, teratogenicity, reproduction toxicity, clinical toxicity, clinical adverse reactions, etc. The functional profile of a chene can include data obtained from any species of organism, including animal (e.g., human), plant, or microorganism, and those obtained at any level of biological system, such as biological molecule, intracellular organ, cell, tissue, organ, and whole body. It is to be noted further that the functional profile of a chene can incorporate data obtained in the presence of one or more different chene(s) in a biological system of interest.

Terms such as "clusters", "families", "species", "orthologs", and "homologs" as used herein are similar in their meaning to those used in genomics and evolutionary genomics. In chenomics, however, these terms are used to express not only similarity and type or closeness of relationship (as used in phylogenic genomics) in the "structure" of two or more chenes, but also similarity and type or closeness of relationship in the "function" of two or more chenes. For example, as to "cluster", distinction in term is achieved by naming one as "structural cluster" and the other as "functional cluster". An example of a structural cluster is a group of chenes which share the same scaffold when combinatorial chemistry technology is used for synthesizing chenes. On the other hand, an example of a functional cluster is a group of chenes which demonstrate identical, similar, or closely related expression profiles (as determined by mRNA levels) in a specified cell (type) or tissue. It is suggested herein that variant, species, cluster, and family are used in an increasing order of population size. Such terms as "ortholog" and "homolog" are conveniently used in chenomics to express the relationship of two or more chenes as obtained by analysis of data according to certain rules that are similar to those used to delineate the phylogenic relationship in evolutionary genomics. For example, when a group of chenes has the same functional group or groups but with slightly different scaffolds in their structures, the relationship between them may be called (structural) orthologs. The use of these words in expressing phylogenic relationship of different functional profiles may become possible when interactive chains of events in the biological system at molecular level are clarified.

Terms such as "pathway", "cascade", "network", and "crosstalk" are used herein to mean the same concepts as commonly used in molecular biology, although "network" and "networking" are used elsewhere for data transmission.

The "information library of chenes" of this invention is particularly useful for identifying candidate compounds (chenes) with desired effects in the process of drug discovery and development. For example, when one desires to select a group of compounds with a specific action on the biological system and places a query in this library with a set of keywords which indicate that specific action, one can obtain a list of the group of compounds having queried action with information of their structural and functional profiles. Examination of the output data will give a distinctive idea about what compounds exhibit the specified action and which other compounds do not. As both structural and functional data are obtained, an efficient process of drug discovery is made possible. Instead of specifying a single action, one can also place an appropriate query to obtain a list of chenes with desired sets of structural and functional profiles. For example, the following query is possible: flavones with high affinity for IL-6 receptor, low cytotoxicity, an appropriate range of drug-likeness parameters, and certain indication for what drug-metabolizing enzyme system. is involved. If one receives a response that there is no such chene in the library, one can relax the query conditions until. one obtains a certain image about what structural requirements will have to be met 'for a desired chene.

As described subsequently, by incorporating appropriate software into the library, it would even become possible to "predict" what chene would most desirably meet queried requirements, what its structure is, and how to synthesize it. This is a surprising advantage of the information library of chenes and this kind of use of the information library of chenes is expected to accelerate tremendously the discovery and development of useful drugs.

### Construction of information library of chenes

The information library of chenes of this invention can be constructed based on internal information belonging to a single individual, a single company, or a single organization or group. In addition, the information library of chenes of this invention can be constructed by integration of information from multiple sectors selected from individuals, companies, organizations, and groups. Furthermore, such information can be publicly known or unknown (i.e., novel).

Much of the information on chemical substances is hidden within the framework of a private enterprise and rarely or only occasionally disclosed publicly. This can pose a formidable restriction on the advancement of science. Even if publicly disclosed, the chemical substance of interest in question may be protected to a degree by intellectual property rights or by a filed patent application on its synthesis, preparation, access, and use.

Particularly, as described previously, information and data relative to those chenes which have been prepared for the purpose of drug research and development are largely buried within the company that has studied them and are rarely publicly disclosed unless a success is achieved. Hence, the opportunities are largely limited for different companies and academic researchers to be able to share such information and data. This is undesirable in view of the foundations and advancement of science.

It is therefore desirable to construct such systems as mentioned below by which concerned parties can share relevant information and data and effectively establish and advance related science (i.e., "chenomics") because of researching on such information and data.

### Data sharing systems

It is necessary to caution here that science is not a monopoly of so-called academia. It is possible to construct and share science at the level of private industries only, if there is a legitimate reason for it. On the other hand, it is also possible to invite academic researchers for collaborations with private industries if certain conditions are met.

There are several reasons for the fact that each company has a tendency of hesitating to publicly disclose information and data obtained in-house. This invention includes practical means to reduce such a tendency by eliminating the causes for such reluctance. With this invention, it is likely that each company and its researchers become willing to submit their information and data, and will enjoy the opportunity of sharing a pool of data that have been collected, accumulated, integrated, and classified. In addition, academic researchers will be pleased to collaborate with the industrial sectors if imposed conditions are acceptable to them.

Furthermore, in this invention, it is possible to add various sub-systems and systems parallel to the original ones. This invention includes each of these added systems and the whole after addition of one or more of selected parallel system(s) and/or sub-system(s). When a set of requirements are defined in a query, an example of such additional system is, as described in the preceding section, one which can predict what chene would most desirably meet queried requirements, what its structure is, and how to synthesize it. This can be called *"in silico* drug design and synthesis by reverse screening." Such a system integrated as a whole is expected to automatically generate novel findings in chenomics as the amount of data and information cumulatively increases with time. In other words, the system of this invention is a system which "does science" on its own and by itself.

With pharmaceutical industry as an example, certain disturbing situations and problems in this industry can be solved via this invention and are described herein. Such situations and problems, and corresponding solutions provided by this invention, however, almost equally apply to other industries which are associated with use and production of the chene. Examples of fields of such other industries include, but not limited to, chemical, food, agricultural, environmental, and health industries. In addition, this invention is expected to be' useful for the information industry and communication industry as it relates to information and communication.

The system of this invention and associated embodiments thereof have not been described nor have been in existence in the past and therefore are novel. The system of this invention and associated embodiments thereof have not been suggested nor proposed in the'past and therefore are a truly novel.

### Brief Description of the Drawings

Fig. 1 illustrates various concepts in chenomics.
Fig. 2 illustrates the fundamental structure of the system for sharing information and data pertaining to chenomics.

### Best Mode to Carrying out the Invention

This invention is specifically illustrated below with reference to Examples, chiefly on data sharing systems; however, it is not construed as being limited thereto.

### [Example 1] Fundamental structure

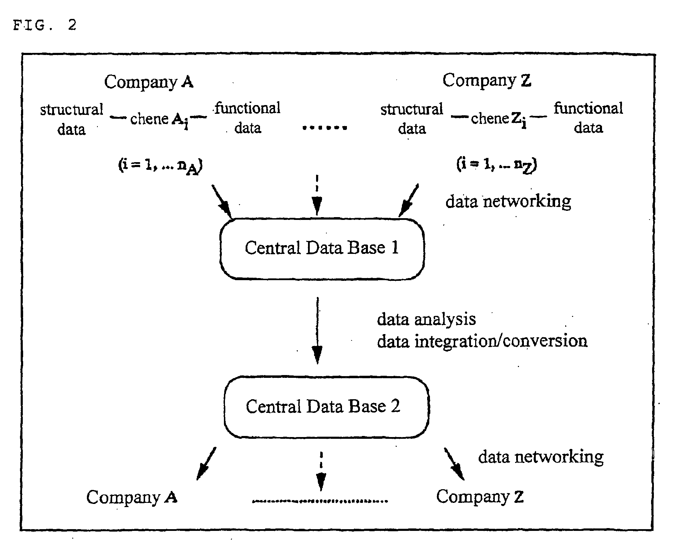

The fundamental structure of this invention is shown in Fig. 2. There can be innumerable structures which are derived from the fundamental structure and by combination thereof. The upper part of Fig. 2 indicates both structural and functional data concerning a plurality of chenes which are possessed by different member companies.. (The term "member" can be used with the term "client".) These data are first sent, for example by closed means and/or systems of communication, to Central Data Base 1 (data networking) and accommodated and stored therein. While the term "member company" is used in Fig. 2, the membership is not limited to a company but is open to an individual or a group formed by specific individuals; for example, individuals belonging to a particular laboratory of a company or academia are also eligible. However, as described subsequently, member company, individual member, and each individual belonging to the member group must observe certain rules for confidentiality. Violation of these rules may be accompanied by a reasonable amount of punishment and cancellation of its, his, or her membership. Each set of the data accommodated in Data Base 1 is examined upon receipt for its reliability automatically by a set of computer programs and/or semi-manually (a set of data with dubious reliability is returned to the originator with appropriate comments). Sets of data passing the receipt examination are analyzed and classified (data analysis) and integrated (data integration) into the pool of already collected data. The process of data analysis and classification utilizes a set of computer programs and systems and includes conversion of data which enables both retrieval of requested data in response to a query by a member and transmission of the requested data to the member. The collected data are converted in this manner, integrated in the pool of already collected data, and accommodated and stored in Central Data Base 2. When a query is received from a member, required information is retrieved from the integrated database stored in the Central Data Base 2 and sent to the member, for example, by a closed means or by systems of communication (data networking). As data and information are accumulated over time, this system becomes increasingly valuable for members who share such data and information. Unless returned on receipt of data for reason of dubious reliability, data once stored in Central Data Bases 1 and 2 should not be destroyed or discarded. As described subsequently, this is because there may arise a need later to determine who submitted a particular set of data first. It is desirable for the system to be equipped with a duplication backup system that is to be activated in case of an unforeseen accident. Similar precaution is to be taken with respect to computer programs. This system is constructed in such a manner as to be inaccessible to outsiders and protected against any attempt to disturb its function by unauthorized person.

### [Example 2] Structures to promote submission and sharing of information; sub-types of the system

The system of this invention is closed, but it is possible to construct various sub-types of the system that are, to various degrees, open to different members. These sub-types are called "semi-closed systems". Since companies are frequently competitive with each other, they usually pay their best attention to avoid leakage of information which results in certain advantage to their competitors. In other words, they do their best to protect their confidential information from leakage. Accordingly, it is understandable that they more or less tend to hesitate to participate in this kind of system. Because disclosure of information is guaranteed to be limited to a defined group of members in a perfectly closed system, they would prefer it to be semi-closed.

The smallest of this system (in terms of the number of members) is the one in which disclosure of information and sharing is limited within a company. In this case, those who want to practice this invention can construct a closed system according to this invention and either sell the same system to all the clients or sell respective customized systems to multiple different clients.

It is possible for two or more companies to form a consortium. For example, companies in different industries that are not competitive in the market may even be willing to participate in such a consortium.

Even among competitive companies, it is possible to form a mini-consortium by limiting the extent of data sharing to a specific cluster or family as defined by either structural chenomics or functional chenomics, or both. The value of this invention lies also in this device to stratify areas of information to enable this kind of limited collaboration. This can be called acceleration of data sharing by "data stratification."

Those who want to practice this invention can construct a closed system that is suitable for a group of different company members and sell it to such a consortium or mini-consortium.

On the other hand, various systems are devised as follows in order to encourage data submission and promote data sharing. One of the means is patent application. Once a patent application is filed based on various attributes of a certain structural cluster or a functional cluster or both of chenes, the legal position of the applicant becomes guaranteed to a degree and thus, for that applicant, it would become easier to submit relevant data-to-databases of this invention. Hence, a person who wants to practice this invention for sale' may encourage patent application filing by members of the consortium or the mini-consortium.

Furthermore, particularly in a closed system, members (including academic members who guarantee defined confidentiality requirements for that closed system) can mutually agree on, receiver's information is guaranteeing to give certain right resulting from the use of the information to the originator of the information who submitted it first. Alternatively, the members may agree to pay a certain amount of money to such an originator. In this case, while a patent application may or may not have been filed on the subject, the amount of guaranteed return either in the form of right or money or both is influenced by the presence or absence of patent application and the contents and value of that patent application. Thus, prior to providing data, the filing of a patent application is highly recommended to increase the value of the information. It is further recommended that members agree on a clause when they are involved in a dispute, so that they can bring their case to a neutral arbitration agency or organization for settlement. This would give a certain amount of easiness and encourage submission and sharing of their data. All these agreements are recommended to be documented and such documentation can be maintained in the system of this invention under strict confidentiality. These measures for agreement are included in the system and the additional embodiments of this invention.

A semi-closed system is formed by imposing different kinds and degrees of restriction on an open system. When such restriction reaches a maximum, the system practically returns to a closed system. An effective measure for such restriction is to "stratify" and/or "hierarchize" data and information accessible to members other than originators. By taking this measure, it becomes possible to allow for non-originator members to have limited access to information and data. The degree and extent of such access is based on the person requiring such access (data seeker) and the amount of guaranteed return in right and/or money. As restriction is strengthened, submission of data and information by members will be accelerated. This and other collateral measures are incorporated into this invention.

Who wants to have access to data and information (data seeker) is a critical factor in granting such access. This can be determined on the basis of whether the data seeker belongs to a company or academia (also, whether seeker-affiliated academic institution is public or private). If the data seeker belongs to a company, what industry it is, number of employees, sales volume, total capital and other financial statements, attained success in relevant area, strength of commitment of development in the field pertaining to the data and information, etc. are all important factors to consider. In addition, it is desirable for the originator to have right and freedom to choose an eligible data seeker to be given such access without duty to disclose to any party the reason for the choice. In addition, the data seeker can have confidential information of his or her own which may benefit the originator directly or indirectly. In such a case, the data seeker can add its value to guaranteed return for receiving information. This may require negotiation, and the scheme of the negotiation as outlined here is incorporated into the form of networking sub-system in this invention.

"Stratification" of data and information has already been explained.

"Hierarchization" of data and information means hierarchization by which the level of disclosure by the originator can be determined. The level of disclosure by the originator can be determined by whether or not the originator is comfortable with that level of disclosure, and further depends, for example, on who seeks access to relevant data and what degree and extent of return is guaranteed. The originator determines this hierarchization. For example, the originator may have in advance classified potential data seekers, graded the value of guaranteed return, and assigned an appropriate stepwise hierarchy to submitted data and information. This process may also require negotiation, and the scheme of the negotiation as outlined here is incorporated into the form of networking sub-system in this invention.

When a data seeker desires to have access to certain integrated information originating from multiple parties, this is to be settled by negotiation with each of these originators. A sub-system can be incorporated to enable prior determination of the value of data at the time of data submission (i.e., numerically according to a certain rule). Reference to this numerical value of that set of data may be able to resolve difficult disputes among multiple members. For example, a proportionate return as determined with reference to such numerical values is expected to settle potential disputes easily. Depreciation can occur in the predetermined value (i) when the corresponding patent application is later published; and/or (ii) when there is a third party who has publicly disclosed the same information prior to publication of the patent application in question. The previously mentioned sub-system also incorporates this aspect.

The reverse auction system first invented for sale of airline tickets (US Patent NO: 5,797,127), if adopted by and incorporated into this invention, is an effective means to improve the value of the system of this invention. If it is incorporated into this invention, a data seeker first defines the requested data, for example, with structural and/or functional cluster, family, and so on and then presents to the system a maximum guaranteed return in exchange for obtaining the requested data. The originator who has submitted the requested data is searched for and identified by the data seeker (in closed system), system administrator (in closed or semi-closed system), or the originator himself or herself. The originator is advised of the data seeker and associated information on the data seeker through the system and make a bid for requested return. The originator who has bid the minimum return gives the data seeker right to look at and use requested data. Particularly, when duplicative data from different originators have been accommodated in the system, this method is expected to promote data submission and sharing.

### [Example 3] Integration of data and information

Data and information are preferably in a predetermined format but this is not an absolute requirement. The data that is received can be converted into the predetermined format by the system with the use of appropriate software and/or semi-manually. The data thus converted are analyzed, classified and integrated so that members can search the pool of previously accumulated data via commercially available software (as each company is doing in-house). For chenes, in particular, for structural chenomics, the CAS method can be employed. For both structural, physical, and chemical data and functional data, the methods of Derwent Information can be followed. Commercially available software such as ISIS Base, ISIS Draw and its improved versions (e.g., that of Molecular Design, Ltd.) are useful for this purpose. As described above, the integrated data is housed and maintained in Central Data Base 2 in Fig. 2.

The system can "do science" of its own by itself. The system of this invention has various incorporated programs for statistical analysis which summarize multivariate relational data and put them into integration according to each of specific purposes. This is equivalent to research for science. For example, the system of this invention can stratify the data on chenes into appropriate clusters or families, can assign certain phylogenic or hierarchical positions to chenes such as ortholog and homoiog according to their structural, physical and chemical data and functional data, and can structure the data in the form of a relationship of pathway, cascade, network, and crosstalk. This can be achieved on both structural, physical and chemical data and functional data, separately, and the results obtained are useful for advancement of science and technology. However, when a correlation is found between certain sets of structural and functional data and further when certain rules are discovered and validated between structure and function, the value of such analyses incorporated into the system attains its maximum. "In silico drug design by reverse screening" previously described comes readily to reality when such maximum is reached.

Examples of the methods for statistical analysis to be incorporated into the system include, multivariate regression analysis, principal component analysis and its variants, canonical correlation analysis, multivariate analysis of variance, discriminant function, and cluster analysis. Corresponding software is available free of charge or commercially available. The Markov Model Method and, particularly, its variant, the Hidden Markov Model Method, have yielded interesting conclusions in the field of genomics in terms of phylogenic analysis and study of similarity of homologous or heterologous genes. These latter methods are thus expected to be particularly useful for the system to "do science" by itself.

### [Example 4] Connection with public or non-public databases and co-integration

Public databases can be connected with and integrated in the system of this invention. In chemical field, CAS-ON-LINE is an example thereof. Examples of genomic and proteomic databases include NCBI, EBI, EMBL, and DDBJ.

Connection with. non-public databases and integration is desirable if terms and conditions are acceptable. Examples include WDI (Derwent Information), Comprehensive Medicinal Chemistry (CMC, MDL Information Systems), and Available Chemicals Directory (ACD, MDL Information Systems),

### Industrial Applicability

This invention serves to study the relationship between all the chemical agents that are (or can be) present in the world, regardless of whether they are known or unknown, and the biological systems and substances belonging to the biological system. Thus, this invention contributes to the advancement of related science and industrial technology. The fields of science this invention advances, include medical science, pharmacology, pharmaceutical science, pharmaceutical chemistry, toxicology, environmental science, agricultural science, and engineering. Industries which benefit from this invention include pharmaceutical industry, health care industry, chemical industry, food industry, pesticide industry, plant industry, environmental industry, information industry, and communication industry.

## Claims

1. A method comprising the steps of:
relating a set of structural data and functional data of a chemical substance that is internal information of a single individual, company, organization, or group;
accommodating said related set of data into a database;
analyzing and classifying said related set of the data;
converting the data into a form which enables the individual, company, organization, or group who has submitted the original set of structural data and functional data, to conveniently search for the required information at a later time; and
accommodating and cumulatively storing the converted data into another database.

2. The method of claim 1, wherein said method comprises the steps of:
analyzing and classifying a set of structural data and functional data of a chemical substance;
converting the data in a form which enables individual, company, organization, or group who has submitted the original set of structural data and functional data, to conveniently search for required information at a later time; and
accommodating and cumulatively storing the converted data into a database.

3. An information library of chemical substances constructed according to the method of claim 1.

4. A system comprising the information library according to claim 3 and means for enabling the individual, company, organization, or group who has submitted the original set of structural data and functional data, to conveniently search for 5 required information at a later time according to claim 1.

5. A method comprising the steps of:
relating a set of structural data and functional data of each chemical substance possessed internally by a multiple sectors selected from the group consisting of multiple individuals, companies, organizations, or groups consisting of multiple individuals;
accommodating the related set of the data into a database;
analyzing and classifying the related set of the data;
converting the data in a form which enables a person to conveniently search for required information at a later time; and
accommodating and cumulatively storing the converted data into another database.

6. A database constructed by the method of claim 1 or 5.

7. A method for relating a set of structural data and functional data of a chemical substance, said method comprising the step of using the method of claim 1 or 5.

8. The method of claim 5, wherein the method comprises the steps of:
analyzing and classifying a set of structural data and functional data of a chemical substance;
converting the data in a form which enables a person to conveniently search for required information at a later time; and
accommodating and cumulatively storing the converted data into a database.

9. An information library of chemical substances constructed according to the method of claim 1 or 5.

10. A system comprising the information library according to claim 9 and means for enabling a person to conveniently search for required information at a later time according to claim 1 or 5.
